# EUROPEAN PATENT SPECIFICATION

(11) **EP 1 853 721 B1**
(45) Date of publication and mention of the grant of the patent: **14.04.2010**
(21) Application number: 06709785.7
(22) Date of filing: 16.02.2006
(51) Int. Cl.: C12Q 1/48, G01N 33/68, G01N 33/574

(54) **METHOD FOR DETERMINING RESPONSIVENESS TO CHK1 INHIBITORS**
VERFAHREN ZUR BESTIMMUNG DER REAKTIONSFÄHIGKEIT GEGENÜBER CHK1-INHIBITOREN
PROCEDE PERMETTANT DE DETERMINER LA SENSIBILITE A DES INHIBITEURS DE LA CHK1

(30) Priority: 18.02.2005 US 654131 P
(43) Date of publication of application: 14.11.2007
(73) Proprietor: AstraZeneca AB, 151 85 Södertälje (SE)
(72) Inventor: ZABLUDOFF, Sonya, AstraZeneca R & D Boston, Waltham, Massachusetts 02451 (US); BERGERON, Louise, AstraZeneca R & D Boston, Waltham, Massachusetts 02451 (US)
(74) Representative: Giles, Allen Frank
(86) International application number: PCT/GB2006/000548
(87) International publication number: WO 2006/087557

(56) References cited:
- WO-A-2004/081008
- LYNE PAUL D ET AL: "Identification of compounds with nanomolar binding affinity for checkpoint kinase-1 using knowledge-based virtual screening" JOURNAL OF MEDICINAL CHEMISTRY, vol. 47, no. 8, 8 April 2004 (2004-04-08), pages 1962-1968, XP002382303 ISSN: 0022-2623
- OKUBO EIJI ET AL: "Negative regulation of mitotic promoting factor by the checkpoint kinase Chk1 in simian virus 40 lytic infection." JOURNAL OF VIROLOGY, vol. 77, no. 2, January 2003 (2003-01), pages 1257-1267, XP002382042 ISSN: 0022-538X
- ZHOU B-B S ET AL: "Targeting the checkpoint kinases: Chemosensitization versus chemoprotection" NATURE REVIEWS CANCER 2004 UNITED KINGDOM, vol. 4, no. 3, 2004, pages 216-225, XP002382043 ISSN: 1474-175X cited in the application

## Description

### Field of the Invention

The present invention relates to the use of a biomarker as an indicator of a therapeutic agent's activity within a test cancer patient. The biomarker of the present invention can be used, for example, to determine when to administer a therapeutic agent or to select a therapeutically effective dose of a therapeutic agent to a patient.

### Background of the Invention

Chemotherapy and radiation exposure are currently the major options for the treatment of cancer, but the utility of both of these approaches is severely limited by drastic adverse effects on normal tissue, and the frequent development of tumor cell resistance. It is therefore highly desirable to improve the efficacy of such treatments in a way that does not increase the toxicity associated with them. One way to achieve this is by the use of specific potentiating agents. Typically, these agents are designed to be used in combination with existing or novel agents.

An individual cell replicates by making an exact copy of its chromosomes, and then segregating these into separate cells. This cycle of DNA replication, chromosome separation and division is regulated by mechanisms within the cell that maintain the order of the steps and ensure that each step is precisely carried out. Key to these processes are the cell cycle checkpoints (Hartwell et al., Science, (1989) 246(4930):629-34) where cells may arrest to ensure DNA repair mechanisms have time to operate prior to continuing through the cycle into mitosis. There are three such checkpoints in the cell cycle - the G1/S checkpoint that is regulated by p53, the S phase and the G2/M checkpoint that is monitored by the Ser/Thr kinase checkpoint kinase 1 (CHK1).

CHK1 is activated through phosphorylation of Ser 317 and/or Ser 345 by ataxia-xelangiectasia (ATR) and/or ataxia-xelangiectasia mutated (ATM) following DNA damage induced by either chemo or radiation therapy (Zhao, H. et al., (2001) Molecular Cell Biology 21, 7239-7249 ). CHK1 in turn phosphorylates numerous substrates including cdc25A, cdc25C, and TLK (tousled like kinase). Phosphorylation of cdc25A and cdc25C by CHK1 leads to their inactivation though degradation and relocalization, respectively. The inactivation of cdc25A and cdc25C then leads to cell cycle arrest in the S and G2 phases of the cell cycle, respectively. In addition to CHK1 mediating cell cycle arrest following DNA damage, there is emerging data from the literature that CHK1 is involved in DNA repair processes as well (Sengupta et al., Journal of Cell Biology, 166,6:801-813, 2004). Furthermore, data has also suggested a putative role for CHK1 in cell division. Specifically CHK1 appears to have a role in regulating entry into mitosis by preventing premature activation of cyclin B-Cdk1 through regulation of cdc25B (Kramer et al., Nature Cell Biology, 6; 9;884-891, 2004) and in the regulation of replication initiation

As the cell cycle arrest induced by these checkpoints is a crucial mechanism by which cells can overcome the damage resulting from radio- or chemotherapy, their abrogation by novel agents should increase the sensitivity of tumor cells to DNA damaging therapies. Additionally, the tumor specific abrogation of the G1/S checkpoint by p53 mutations in the majority of tumors can be exploited to provide tumor selective agents. One approach to the design of chemosensitizers that abrogate the G2/M checkpoint is to develop inhibitors of the key G2/M regulatory kinase CHK1, and this approach has been shown to work in a number of proof of concept studies. (Koniaras et al., Oncogene, 2001, 20:7453; Luo et al., Neoplasia, 2001, 3:411; Busby et al., Cancer Res., 2000, 60:2108; Jackson et al., Cancer Res., 2000, 60:566).

A biomarker is a characteristic that is objectively measured and evaluated as an indicator of normal biological processes, pathogenic processes, or pharmacological responses to a therapeutic intervention (Atkinson et al, Clin. Pharmacol. Ther., 69: 89-95 (2001)). It is believed that the development of new validated biomarkers will lead both to significant reductions in healthcare and drug development costs and to significant improvements in treatment for a wide variety of diseases and conditions.

In order to optimally design clinical trials and to gain the most information from these trials, a biomarker is required. The marker needs to be measurable in surrogate and tumor tissues and should correlate with the inhibition of the pathway. In addition the biomarker should demonstrate activation of the upstream pathway and inactivation of the downstream pathway. Ideally these markers will also correlate with efficacy and thus could ultimately be used for patient selection.

### Summary of the Invention

The present invention is based, in part, on methods that can be used to determine a patient's responsiveness to a CHK1 inhibitor including determining whether to administer a CHK1 inhibitor, when to administer a CHK1 inhibitor, and to determine a therapeutically effective dose of a CHK1 inhibitor. Specifically the methods of the present invention include the determination of one or more activated CHK1 signal transduction molecules such as phosporylated CHK1. The presence of an activated CHK1 signal transduction molecule indicates that a CHK1 inhibitor should be administered. In another method of the invention, the method includes the determination for the presence of phosphorylated H2AX as an indicator of whether an appropriate dose of a CHK1 inhibitor has been administered to a patient.

The biomarkers of the present invention can be used in the treatment or prophylaxis of neoplastic diseases such as cervical cancer, cancer of the head and neck, carcinoma of the breast, ovary, lung (non small cell), pancreas, esophageal, gastric, small bowel, hepatic, colon, prostate or other tissues, as well as leukemias and lymphomas, including chronic lymphocytic leukemia, tumors of the central and peripheral nervous system, and other tumor types such as melanoma, and sarcomas including fibrosarcoma, mesothelioma, and osteosarcoma, and endocrine tumors such as islet cell tumors and thyroid tumors .

Accordingly, the invention provides a method according to the claims.

The sample of the invention can be any appropriate sample such as a tumor, peripheral blood, a cell scraping, a hair follicle, a skin punch or a buccal sample.

The activated CHK signal transduction pathway molecule can be phosphorylated CHK1 or phosphorylated H2AX. The phosphorylated CHK1 can be determined by a CHK1 antibody, for example, a phophospecific antibody.

### Brief description of the drawings

Fig. 1 depicts a bar chart showing that gemcitabine (Gem) results in the phosphoylation of CHK1.
Figs. 2A-B depicts a bar chart showing phosphorylation of CHK1 following combination treatment of gemcitabine (Gem) and CHK inhibitor, and gemcitabine alone.
Fig. 3 depicts a bar chart showing phosphorylation of CHK1 from tumor sections of H460 Xenograft mice following treatment with gemcitabine (Gem), Chk inhibitor (Chki), and a combination of both.
Fig. 4 depicts a bar chart showing quantitation of phosphorylation of CHK1 in hair follicles from mouse skin biopsies - following treatment with gemcitabine (Gem), Chk inhibitor (Chki), and a combination of both.
Fig. 5 depicts a bar chart showing 53BP1 quantitation on Hela Cells with different amounts of Doxorubicin hydrochloride (Dox) and a CHK1 inhibitor (Chki).
Fig. 6 depicts a bar chart showing phospho-H2AX quantitation of SW620 cells cells treated with gemcitabine (Gem) and a CHK inhibitor (Chki).
Fig. 7 depicts a bar chart showing %phospho-H2AX positive cells when cells are treated with varying amounts of gemcitabine (Gem) and a CHK inhibitor (Chki).
Fig. 8 depicts a bar chart showing %phospho-H2AX positive cells when cells are treated with the combination of Doxorubicin hydrochloride (dox) and a CHK inhibitor.

### Detailed Description

The present invention provides biomarkers to predict a patient's response to a CHK1 inhibitor. The predictive biomarkers can be used to indicate, for example, when to administer a CHK1 inhibitor or to determine the amount of a CHK1 inhibitor which can be used to effectively treat a cancer patient.

### Biomarkers

The present application discloses a number of different biomarkers that can be used to predict a patients responsiveness to a CHK1 inhibitor. The biomarkers of the invention include activated CHK1 signal transduction molecules. An activated CHK1 signal transduction molecule is any molecule that is directly or indirectly modified, for example activated or deactivated, as a result of the activation of CHK1. For example, an activated CHK1 signal transduction molecule can be phosphorylated CHK1. Other molecules that serve as an activated CHK1 signal transduction molecule include phosphorylated TLK1/2, CDC25, cyclin B, Aurora B or phosphohistone H3. In addition, a CHK1 signal transduction pathway molecule includes a molecule that is activated by ATR or ATM such as H2AX.

In one example, the biomarker is H2AX. The presence of phosphorylated H2AX (also known as gamma H2AX) following the administration of both a DNA damaging agent and a CHK inhibitor compared to a control (a sample taken from the patient prior to the administration of CHK1 and a DNA damaging agent or prior to the administration of a DNA damaging agent) is indicative that an appropriate dose of a CHK1 inhibitor has been administered.

### Assays

The present invention provides a number of biomarkers that can be used to predict a patient's responsiveness to a CHK1 inhibitor. One exemplary method for detecting the presence of a biomarker includes obtaining a tumor or surrogate tissue sample from a test subject in the absence of treatment with a DNA damaging agent or cellular stress inducing agent and determining for the presence of the biomarker. In another exemplary method, the method for detecting the presence of a biomarker includes obtaining a tumor or surrogate tissue sample from a test subject who has received a DNA damaging agent and determining for the presence of the biomarker.

### DNA Damaging agent

Typically, a DNA-damaging agent is administered to a test cancer patient. A DNA damaging agent is an extremely toxic agent inducing, for example, double-stranded DNA breakage. While not wishing to be bound by theory, it is believed that once a sufficient amount of a DNA damaging agent has been administered to a cancer patient, cells react by activating DNA damage response pathways including activating cell-cycle checkpoints. It is believed that the cell-cycle checkpoint kinase, CHK1, arrests cells thus allowing repair of DNA and its inhibition leads to tumor cells that are sensitized to cancer agents.

DNA damaging agents that can be used in the present invention include any appropriate DNA damaging agent. Examples of DNA damaging agents include alkylating agents such as cyclophosphamide; other agents that produce single or double stranded DNA breaks such as the platining agents, including cisplatin, carboplatin and oxaliplatin; anti-metabolites such as 5-fluorouracil, fludarabine and gemcitabine; topoisomerase I or III inhibitors such as doxorubicin, etoposide and topotecan; and stress inducing agents such as antibodies to cell surface proteins such as rituximab, cetuximab or trastuzumab, radiomimetics such as bleomycin and gamma radiation.

### Samples

Any appropriate sample can be used to determine for the presence of a biomarker of the invention. In one example the sample is a surrogate tissue sample, for example, the sample can be peripheral blood, saliva, a cell scraping, a buccal sample, hair follicle, skin punch or sputum.

Alternatively, tumor samples can be taken from any patient receiving a DNA damaging agent. For example, the tumor can be a non-solid tumor such as leukaemia, multiple myeloma or lymphoma, or can be a solid tumor, for example bile duct, bone, bladder, brain/CNS, breast, ovary, small bowel, colorectal, cervical, endometrial, gastric, head and neck, hepatic, lung, muscle, neuronal, oesophageal, ovarian, pancreatic, pleural/peritoneal membranes, prostate, renal, skin, testicular, thyroid, uterine, vulval tumors and other tumor types such as melanoma, and sarcomas including fibrosarcoma, mesothelioma, and osteosarcoma, and endocrine tumors such as islet cell tumors and thyroid tumors

### Detection of an activated CHK1 signal transduction pathway molecule

In one example, the method includes determining from a sample of a test patient for the presence of an activated CHK1 signal transduction pathway molecule. In another example, the method includes determining from a sample of a test patient who is being treated with a DNA damaging agent for the presence of an activated CHK1 signal transduction pathway molecule. The presence of an activated CHK1 signal transduction pathway molecule such as phosphorylated-CHK1 or phosphorylated H2AX is determined.

Applicants have determined that following administration of a CHK1 inhibitor it is possible to predict if a CHK1 inhibitor is inhibiting its molecular target. The method includes taking a second sample from the test patient who has received a CHK1 inhibitor and determining for phosporylated CHK1. Typically the sample is taken 4 to 24 hours after the administration of the CHK1 inhibitor. Patients who have received a therapeutically effective dose of a CHK1 inhibitor will have an increased amount (for example, at least a two fold) of phosphorylated CHK1 for a longer period of time, e.g., over a 24 hour period, compared to the amount of phosphorylated CHK1 determined from the first sample. While not wishing to be bound by theory, Applicant's believe that the increased amount of phosphorylated CHK1 following administration of a CHK1 inhibitor is because following the inhibition of molecules downstream of CHK, cells attempt to compensate by increasing activation of molecules upstream of CHK1.

Determining for the presence of an activated CHK1 signal transduction pathway molecule of the invention can be performed using any method known in the art. For example, phosphorylated-CHK1 or phosphorylated H2AX can be visualized by reacting the proteins with antibodies such as monoclonal antibodies directed against the phosphorylated serine, threonine or tyrosine amino acids that are present in the proteins. For example, monoclonal antibodies useful for isolating and identifying phosphotyrosine-containing proteins are described in U.S. Pat. No. 4,543,439.

The procedures and reagents needed to determine an activated CHK1 protein can be readily performed by one of skill in the art. An agent of interest that can be used to detect an activated CHK1 signal transduction molecule includes any molecule such as a peptidomimetic, protein, peptide, nucleic acid, small molecule, an antibody or other drug candidate, that can bind the protein. In one example, antibodies that are commercially available can be used to detect an activated CHK1 signal transduction molecule. For example, antibodies against phosphorylated CHK1 and CDC2 are available from Cell Signaling (Beverly, MA), antibodies against phosphorylated CDC25A Ab-3 are available from Lab-vision (Fremont, CA); antibodies against Aurora B are available from Abeam (Cambridge, MA); antibodies against phosphorylated H2AX are available from Upstate Biotechnology (Upstate, NY).

Typically, antibodies used for visualizing the activated CHK1 signal transduction molecule can be labeled by any procedure known in the art, for example, using a reporter molecule. A reporter molecule, as used herein, is a molecule which provides an analytically identifiable signal allowing one of skill in the art to identify when an antibody has bound to a protein that it is directed against. Detection may be either qualitative or quantitative. Commonly used reporter molecules include fluorophores, enzymes, biotin, chemiluminescent molecules, bioluminescent molecules, digoxigenin, avidin, streptavidin or radioisotopes. Commonly used enzymes include horseradish peroxidase, alkaline phosphatase, glucose oxidase and beta-galactosidase, among others. The substrates to be used with these enzymes are generally chosen for the production, upon hydrolysis by the corresponding enzyme, of a detectable color change. For example, p-nitrophenyl phosphate is suitable for use with alkaline phosphatase reporter molecules; for horseradish peroxidase, 1,2-phenylenediamine, 5-aminosalicylic acid or toluidine are commonly used. Incorporation of a reporter molecule onto an antibody can be by any method known to the skilled artisan.

After separation and visualizing the proteins, the amount of each protein species may be assessed by readily available procedures. For example, by using Western blot analysis which includes electrophoretically separating proteins on a polyacrylamide gel, and after detecting the separated proteins, the relative amount of each protein can be quantified by assessing its optical density. Alternatively, other methods such as FACS, immunohistochemistry, ELISA, etc., can be used.

In the methods of the invention one or more of the activated CHK1 signal transduction pathway molecules can be detected. For example, an assay system can be set up which can detect for the presence of multiple activated CHK1 signal transduction pathway molecules.

### Detection of phosphorylated H2AX

The method of the present invention also includes identifying for the presence of phosphorylated H2AX (also known as gamma H2AX) following the administration of both a DNA damaging agent and a CHK1 inhibitor to a test cancer patient. In choosing the DNA damaging agent, the ability of the DNA damaging agent to phosphorylate H2AX in the presence or absence of a CHK1 inhibitor must have been previously determined. In one example, the method of the invention uses a DNA damaging agent which if administered alone without CHK1 results in a modest or no increase in the phosphorylation of H2AX. Examples of such DNA damaging agents include Gemcitabine and dox. In this example, the combination of the Chk 1 inhibitor and the DNA damaging agent produces a large increase, e.g., a synergistic response, in the phosphorylation of H2AX. According to the method of the invention, an increase in the presence of phosphorylated H2AX compared to a control (the control can be a sample taken from the patient prior to the administration of the DNA damaging agent and CHK1 inhibitor) indicates that the CHK1 inhibitor has been administered at a therapeutically effective dose.

Phosphorylated H2AX can be detected using any agent such as a peptidomimetic, protein, peptide, nucleic acid, small molecule, an antibody or other drug candidate, that can bind the protein. Antibodies that bind phosporylated H2AX are known in the art, for example, antibodies against phosphorylated H2AX. are available from from from Upstate Biotechnology (Upstate, NY).

### CHK1 inhibitors

CHK1 inhibitors are known in the art, for example, the CHK1 inhibitor can include, for example, a peptide, an antibody, an antisense molecule or a small molecule. CHK1 inhibitors useful in the present invention include but are not limited to, those described or claimed in the following publications the entire disclosures of which are incorporated by reference herein. Examples of small molecule CHK1 inhibitors include 2-ureidothiophene compounds, which are described in WO03029241 3-ureidothiophene compounds, described in W003028731 and Chkl inhibitor XL844 (Exilixis, CA). Other CHK1 inhibitors are described in PCT/GB2004/005400, US 20050256157, US 20050245563, US 20050245525, WO 2002070494, US 20040014765 and US 2003199511. Antisense CHK1 oligonucleotides are described in WO 200157206 and US Patent 6,211,164. Interfering CHK1 nucleic acid (siNA) and short interfering RNA (siRNA) are described in US 20050196765.

### Examples

### Example 1: Demonstration of activation of CHK1 following DNA damage in tumor cell lineS, and Xenograft samples

### A. In vitro studies:

In brief, 3 X 10⁶ SW620 and or HT29 cells were plated into 10 cm tissue culture dishes and allowed to adhere for 24 hours at 37°C. Cell were then treated with a titration of DNA damaging agents (SN-38 (CQ International, China), doxorubicin (Sigma-Aldrich, MO) or gemcitabine (CQ International, China)). Cells were then harvested after an 8 hour incubation with DNA damaging agents in Onyx lysis buffer with protease and phosphatase inhibitors (20mM Tris, 137 mM Na Cl, 1mM EGTA, 1% Triton X, 10% glycerol, 1.5 mM MgC12, 1:100 dilution of Calbiochem Phophatase Inhibitor Cocktail set II, 10 mM β-glycerophosphate, 1 mM DTT, 7 µg/ml PMSF, 20KIU/ml aprotinin, 1 mM Pefabloc, 0.1 mg/ml leupeptin). Protein lysates were then generated, protein concentration determined and equal protein loaded and analyzed by Western blot analysis using a 1:100 dilution of phospho-CHK rabbit monoclonal antibody (Ser 345) (133D3) acquired from Cell Signaling (Beverly, MA). Western blot analysis demonstrated a dose dependent up-regulation of phospho-CHK following DNA damage. To further characterize the CHK1 activation, SW620 and HT29 cells were treated with gemcitabine (100 nM) or SN38 (100 nM) and cells harvested at different times (from 30 minutes to 24 hours). Western blot analysis demonstrated a time dependent activation of phospho-CHK with peak activation between 4 and 8 hours. A panel of tumor cell lines was then examined to determine how broadly in tumor cells CHK was activated and whether p53 status impacted CHK activation. The panel of cell lines were plated and treated for 8 hours with 100 nM of gemcitabine. Cell lysates were harvested and evaluated for phospho-CHK upregulation. Western blot analysis demonstrated that phospho-CHK is upregulated in a panel of tumor cell lines, albeit to different levels.

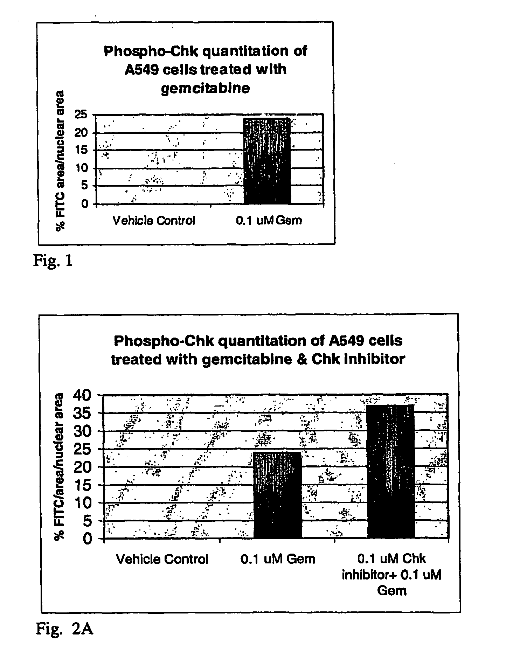

Phospho-CHK regulation was also examined by immunohistochemistry (IHC). In brief, HeLa or A549 cells were plated on cover slips and allowed to adhere for 24 hours at 37°C. Cells were then treated for 5 hours with 100nM of gemcitabine. Cells were fixed with 4% PFA in PBS (8 minutes room temperature), permeabilized with 0.5% Triton-X in PBS, washed with PBS and washed with 0.05% Tween 20 in PBS. Cells were then blocked with 10% Goat serum for 1 hour at room temperature, incubated with 1:50 dilution of phospho-CHK rabbit monoclonal antibody (Ser 345) (133D3) acquired from Cell Signaling (Beverly, MA) in 10% goat serum for 90 minutes, washed twice with PBS and once with 05% Tween 20 in PBS. Cells were then incubated for 60 minutes with 1:300 dilution of Alexa Fluor 488 anti-rabbit IgG (Molecular Probes, OR) in 10% goat serum, washed twice with PBS, nuclei stained with Hoescht solution (2/µg/ml) for 5 minutes and cells rinsed with PBS. Phospho-CHK staining was then quantitated using Metamorph analysis (Universal Imaging Corp, Dowington, PA). Area of phospho-CHK signal relative to nuclear area was measured as shown in Fig. 1. Similar results were obtained using intensity measurements. Data demonstrates a large increase in phopho-CHK following gemcitabine treatment.

### B. In vivo studies:

1 X 10⁷ H460 cells expressing dominant negative p53 were injected into the right flank of male nude rats which had been irradiated with 5 Gy radiation 6 days prior to implantation. At day 20 when the tumors reached a size of 8 grams, animals were dosed IV with 20 mg/kg gemcitabine in 0.9% saline. Tumors were then harvested at varying time points including 1.5, 2, 2.5, 4, 6, 8 and 24 hours following gemcitabine dosing. A section of the tumor was then homogenized in Swedish lysis buffer (20mM Tris pH 8.0, 150mM NaCl, 1% NP-40 substitution, and 1mM Sodium Vanadate with protease inhibitors as recommended (Roche Applied Science, IN) and Phosphotase Inhibitors I and II as recommended (Sigma-Aldrich, MO). Protein lysates were then generated, protein concentration determined and equal protein loaded and analyzed by Western blot analysis using a phospho-CHK rabbit monoclonal antibody (Ser 345) (133D3) acquired from Cell Signaling (Beverly, MA). Western blot analysis demonstrated activation of CHK by 2 hours with a maximum activation at 8 hours. By 24 hours phospho-CHK is significantly down-regulated.

### Example 2: Demonstration of increased phospho-CHKfollowing combination treatment of gemcitabine and CHK1 inhibitor in tumor cell lines, Xenograft samples and surrogate tissues

### A. In vitro studies

In brief, 3 X 10⁶ SW620 were plated into 10 cm tissue culture dishes and allowed to adhere for 24 hours at 37°C. Cell were then treated with 100nM gemcitabine or 30 nM,100 nM or 500 nM CHK inhibitor or a combination of 100nM gemcitabine and 30 nM, 100 nM or 500 nM for 8 hours. One set of cells were then harvested for Western blot analysis. Media containing compounds were removed from the remaining cells and fresh tissue culture media added back. Cells were then harvested 22 hours later, protein lysates generated as described above and analyzed by Western blot analysis using the phosphor-CHK antibody. Western blot analysis demonstrated an increase in phospho-CHK following gemcitabine treatment alone and a dose dependent increase over gemcitabine alone in phospho-CHK following the combination treatment. No increase in phospho-CHK was observed following treatment of cells with CHK inhibitor alone.

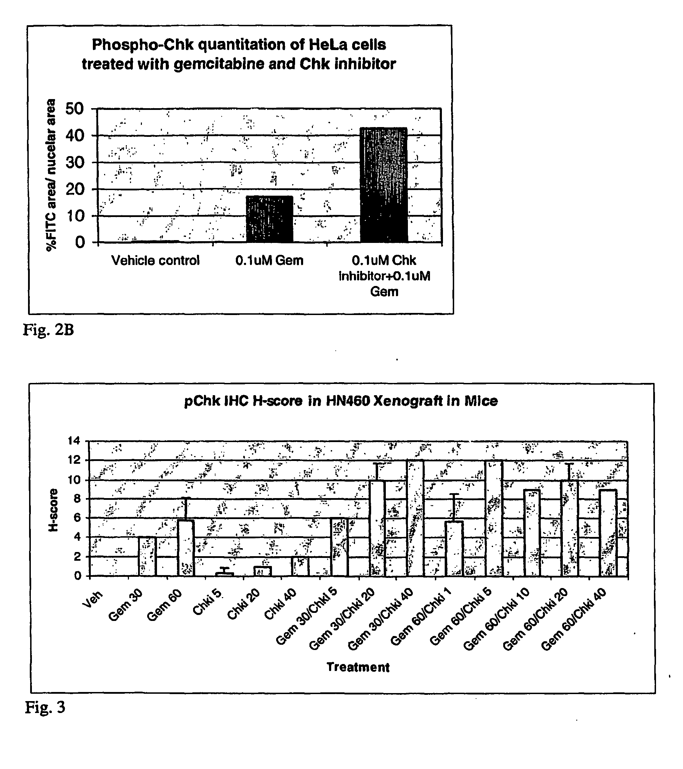

Phospho-CHK regulation was also examined by IHC as described above in Example 1 using HeLa and A549 cells. Fig. 2A and 2B demonstrates a larger increase in phospho-CHK following combination treatment of gemcitabine and CHK inhibitor as compared to gemcitabine alone.

### B. In vivo studies

3 X 10⁶ H460 cells expressing dominant negative p53 were injected into the right flank of male nude mice. At day 25 when the tumors reached a size of 0.6 - 0.8 grams, animals were dosed IV with gemcitabine (30 or 60 mg/kg), Chk inhibitor (5, 20 or 40 mg/kg) or the combination. Seven hours later, tumours were then harvested, fixed and stained for phosphorylated CHK1 (Cell Signalling 2345)-DAB with methyl green counterstain. Sections were then quantitated by H-score analysis. H score is calculated by scoring the intensity of the cells (0, 1, 2 or 3) and multiplying by the score representing the number of positive cells (0, 1 (1-25%), 2 (26-50%), 3 (51-75%) and 4 (76-100%). The analysis shown in Figure 3 demonstrated an increase in the phospho-Chk staining as compared to either agent alone. Similar combination studies were completed with irinotecan and Chk inhibitor. Again data demonstrated an increase in phospho-Chk staining as compared to either agent alone (data not shown).

### C. Surrogate tissues

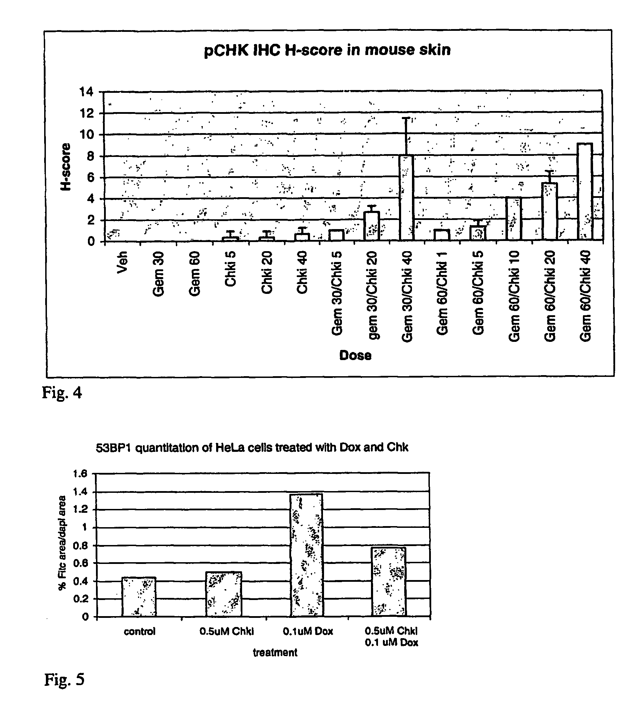

Nude mice were dosed IV with gemcitabine (30 or 60 mg/kg), Chk inhibitor (5, 20 or 40 mg/kg) or the combination as in the section above. Seven hours later, skin biopsies containing hair follicles were then harvested, fixed and stained for phosphorylated CHK1 (Cell Signalling 2345)-DAB with methyl green counterstain. Results showed positive staining (brown) in hair follicle basal cells in treated mice. Staining was specific to cell nuclei. Rare or occasional staining occurred in Chk inhibitor treated groups. No staining was observe in the gemcitabine treated groups. Sections were then quantitated by H-score analysis. H score is calculated by scoring the intensity of the cells (0, 1, 2 or 3) and multiplying by the score representing the number of positive cells (0, 1 (1-25%), 2 (26-50%), 3 (51-75%) and 4 (76-100%). The analysis shown in Figure 4 demonstrates staining of hair follicles is greatly increased in the combination groups compared to the other treatment groups. In addition a dose response of the Chk inhibitor was observed with the largest increases reached at efficious doses.

Similar combination studies were completed with irinotecan and Chk inhibitor. Again data demonstrated an increase in phospho-Chk staining as compared to either agent alone (data not shown).

### Example 3: Demonstration of decreased DNA damage foci following inhibition of CHK1 kinase

### A. In vitro studies:

HeLa cells were plated on cover slips and allowed to adhere for 24 hours at 37°C. Cells were then treated for 5 hours with 100 nM Adriamycin™ and 500 nM CHK inhibitor. Cells were then treated as described in Example 1. Primary antibody used for the studies was a 1:120 dilution of rabbit polyclonal anti-53BP1 (Novus, CO) and secondary antibody was a 1:300 dilution of Alexa Fluor 488 anti-rabbit IgG (Molecular Probes, OR). Foci were quantiatated by measuring fluorescent intensity per number of nuclei (determined by Hoescht staining) using Metaphorph analysis. Figure 5 demonstrates a decrease in the fluorescent intensity when CHK inhibitor is combined with Doxorubicin hydrochloride (Adriamycim™).

### Example 4: Demonstration of increased phospho-H2AX following combination treatment of gemciabine and CHK1 inhibition

### A. In vitro studies

In brief, 3 X 10⁶ SW620 and or A549 cells were plated into 10 cm tissue culture dishes and allowed to adhere for 24 hours at 37°C. Cell were then treated with vehicle, 100 nM gemcitabine, or 100 nM gemcitabine and 500 nM CHK inhibitor. Cells were then harvested after a 5 hour incubation directly into SDS sample buffer (NuPAGE #NP007). Samples were then sonicated, passed through a 21 gauge needle, heated to 100°C for 5 minutes, microcentrifuged and analyzed by Western blot analysis. Protein lysates were then generated, protein concentration determined and equal protein loaded and analyzed by Western blot analysis using a 1:5000 dilution of a mouse monoclonal anti-phospho H2AX Serine 139 (Upstate, NY). Data demonstrated a large increase in phospho-H2AX when cells were treated in combination with gemcitabine and CHK inhibitor and no change or a very modest change when either compound was used alone.

Phospho-H2AX was also examined by a quantitative ELISA assay. In brief, 3 X 10⁶ SW620 were plated into 10 cm tissue culture dishes and allowed to adhere for 24 hours at 37°C. Cell were then treated with vehicle, 100 nM gemcitabine, 30, 50, 100 and 500 nM Chk inhibitor or the combination of gemcitabine and all Chk inhibitor concentrations for 7 hours and then harvested in lysis buffer (20 mM Tris HCL, 28mM Tris base, 0.4%LDS, 2%Glycerol, 0.10 mM EDTA) containing phosphatase inhibitors (Calbiochem Cat. #534625, CA).

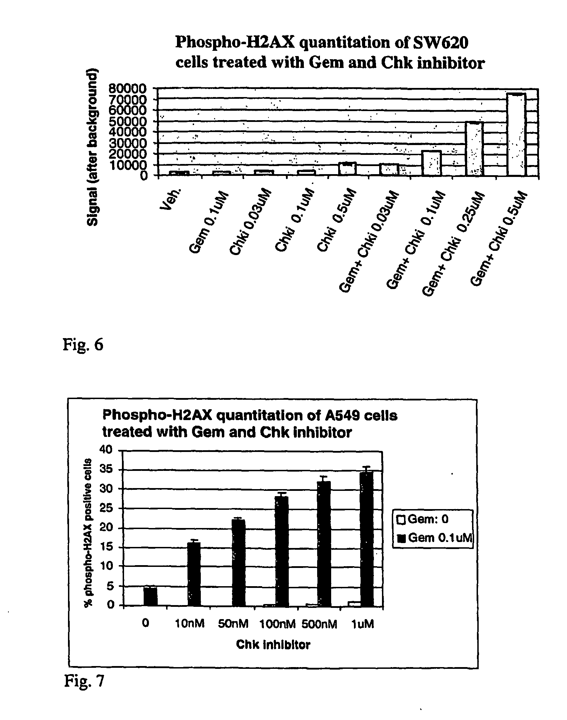

Protein concentrations were determined and p-H2AX levels quantitated by ELISA assay using total H2AX antibody (Novus Biologicals, Cat #NB100-383, CO) to capture total H2AX and LANCE Eu-labeled anti-phospho-histone H2AX (Ser 139) antibody (Upstate, Cat#05-636, NY) to detect the phosphorylated H2AX. As shown in Figure 6, a large increase in phospho-H2AX was observed in the combination treatment.

Phospho-H2AX was also examined by IHC as described above in Example 1 except with the following changes. HeLa or A549 cells were plated into 96 well plates and treated with or without 100 nM gemcitabine and increasing concentrations of CHK inhibitor (0 to 1 µM) for 5 hours prior to fixation and staining. A 1:400 dilution of a mouse monoclonal anti-phospho H2AX Serine 139 (Upstate, NY) followed by a 1:300 dilution of Alexa Fluor 594 anti-mouse IgG (Molecular Probes, OR) was incubated on fixed cells. Quantitation was performed using Cellomics Cell Health algorithms and reported as %phospho-H2AX positive cells. Fig. 7 shows the quantitation demonstrating a robust dose dependent increase in %phospho-H2AX positive cells when cells are treated with the combination of gemcitabine and CHK inhibitor. It was observed using immunostaining that phosphorylated H2AX is present throughout the nucleus, not limited to foci.

### Example 5: Demonstration of increased phospho-H2AXfollowing combination treatment of doxorubicin and CHK1 inhibition

### In vitro studies

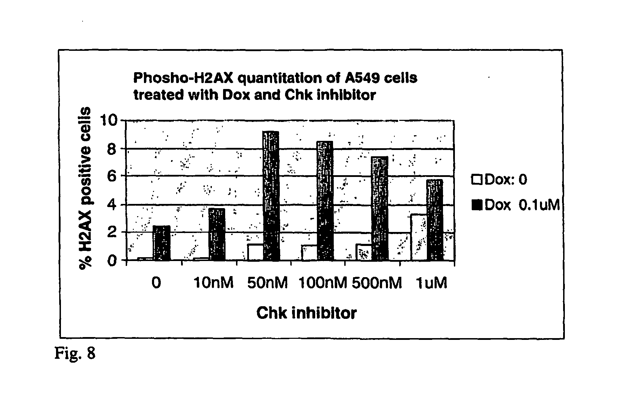

Phospho-H2AX was also examined by IHC as described above in Example 1 except with the following changes. HeLa or A549 cells were treated with or without 100 nM doxorubicin (Adriamycin™) and increasing concentrations of CHK inhibitor (0 to 1 µM) for 5 hours prior to fixation and staining. A 1:400 dilution of a mouse monoclonal anti-phospho H2AX Serine 139 (Upstate, NY) followed by a 1:300 dilution of Alexa Fluor 594 anti-mouse IgG (Molecular Probes, OR) was incubated on fixed cells. Quantitation was performed using Cellomics Cell Health algorithms and reported as %phosphor-H2AX positive cells. Fig. 8 is the quantitation demonstrating a robust dose dependent increase in %phospho-H2AX positive A549 cells when cells are treated with the combination of Adriamycin™ and CHK inhibitor.

## Claims

1. A method for determining if a CHK-1 inhibitor is inhibiting its molecular target CHK-1, the method comprising:
(a) determining for the presence of a phosphorylated CHK-1 molecule in a first sample from a test cancer patient who has received a DNA damaging agent, and
(b) determining for the presence of a phosphorylated CHK-1 in a second sample from the test cancer patient who has received a DNA damaging agent and a CHK-1 inhibitor, wherein an increase in the amount of phosphorylated CHK-1 in the second sample compared to the first sample is indicative that the CHK-1 inhibitor is inhibiting its molecular target CHK-1.

2. The method of claim 1, wherein the sample is a tumor, peripheral blood, a cell scraping, a hair follicle, a skin punch or a buccal sample.

3. The method of any one of claims 1-2, wherein the phosphorylated CHK1 is determined by a CHK1 antibody.

4. The method of any one of claims 1-3, wherein the phosphorylated CHK1 is phosphorylated at Ser 345.

5. A method for determining if a CHK-1 inhibitor is inhibiting its molecular target CHK-1, the method comprising:
(a) determining for the presence of phosphorylated H2AX in a first sample from a test cancer patient who has received a DNA damaging agent; and
(b) determining for the presence of phosphorylated H2AX in a second sample from the test cancer patient who has received a DNA damaging agent and a CHK-1 inhibitor, wherein an increase in the presence of phosphorylated H2AX in the second sample compared to the first sample is indicative that a CHK1 inhibitor is inhibiting its molecular target CHK-1.

6. The method of claim 5, wherein the sample is a blood sample with circulating tumour cells.

7. The method of any one of the preceding claims, wherein the cancer is a non-solid tumor or a solid tumor.

8. The method of claim 7, wherein the tumor is leukemia, multiple myeloma or lymphoma.

9. The method of any one of the preceding claims, wherein the DNA damaging agent is an alkylating agent, an anti-metabolite, a topoisomerase inhibitor, a radiomimetic or gamma radiation.

10. The method of claim 9, wherein the anti-metabolite is 5-fluorouracil, fludarabine or gemcitabine.

## Patentansprüche

1. Verfahren zur Bestimmung davon, ob ein CHK-1-Inhibitor sein molekulares Ziel CHK-1 inhibiert, wobei man in dem Verfahren:
(a) eine Bestimmung auf das Vorhandensein eines phosphorylierten CHK-1-Moleküls in einer ersten Probe aus einem Test-Krebspatienten, der ein DNA-Schäden verursachendes Agens erhalten hat, und
(b) eine Bestimmung auf das Vorhandensein einer phosphorylierten CHK-1 in einer zweiten Probe aus dem Test-Krebspatienten, der ein DNA-Schäden verursachendes Agens und einen CHK-1-Inhibitor erhalten hat, durchführt, wobei ein Anstieg der Menge an phosphorylierter CHK-1 in der zweiten Probe im Vergleich zur ersten Probe darauf hindeutet, dass der CHK-1-Inhibitor sein molekulares Ziel CHK-1 inhibiert.

2. Verfahren nach Anspruch 1, wobei es sich bei der Probe um einen Tumor, peripheres Blut, einen Zellabstrich, ein Haarfollikel, ein Haut-Stanzbiopsat oder eine Bukkalprobe handelt.

3. Verfahren nach einem der Ansprüche 1-2, wobei die phosphorylierte CHK1 mit einem CHK1-Antikörper bestimmt wird.

4. Verfahren nach einem der Ansprüche 1-3, wobei die phosphorylierte CHK1 an Ser 345 phosphoryliert ist.

5. Verfahren zur Bestimmung davon, ob ein CHK-1-Inhibitor sein molekulares Ziel CHK-1 inhibiert, wobei man in dem Verfahren:
(a) eine Bestimmung auf das Vorhandensein von phosphoryliertem H2AX in einer ersten Probe aus einem Test-Krebspatienten, der ein DNA-Schäden verursachendes Agens erhalten hat, und
(b) eine Bestimmung auf das Vorhandensein von phosphoryliertem H2AX in einer zweiten Probe aus dem Test-Krebspatienten, der ein DNA-Schäden verursachendes Agens und einen CHK-1-Inhibitor erhalten hat, durchführt, wobei ein erhöhtes Vorhandensein von phosphoryliertem H2AX in der zweiten Probe im Vergleich zur ersten Probe darauf hindeutet, dass ein CHK1-Inhibitor sein molekulares Ziel CHK-1 inhibiert.

6. Verfahren nach Anspruch 5, wobei es sich bei der Probe um eine Blutprobe mit zirkulierenden Tumorzellen handelt.

7. Verfahren nach einem der vorhergehenden Ansprüche, wobei es sich bei der Krebserkrankung um einen nicht soliden oder einen soliden Tumor handelt.

8. Verfahren nach Anspruch 7, wobei es sich bei dem Tumor um Leukämie, multiples Myelom oder Lymphom handelt.

9. Verfahren nach einem der vorhergehenden Ansprüche, wobei es sich bei dem DNA-Schäden verursachenden Agens um ein Alkylierungsmittel, einen Antimetaboliten, einen Topoisomerase-Inhibitor, ein Radiomimetikum oder um Gammastrahlung handelt.

10. Verfahren nach Anspruch 9, wobei es sich bei dem Antimetaboliten um 5-Fluoruracil, Fludarabin oder Gemcitabin handelt.

## Revendications

1. Méthode pour déterminer si un inhibiteur de CHK-1 inhibe sa cible moléculaire CHK-1, la méthode comprenant:
(a) la détermination de la présence d'une molécule de CHK-1 phosphorylée dans un premier échantillon provenant d'un patient atteint de cancer à tester qui a reçu un agent endommageant l'ADN, et
(b) la détermination de la présence d'une CHK-1 phosphorylée dans un deuxième échantillon provenant d'un patient atteint de cancer à tester qui a reçu un agent endommageant l'ADN et un inhibiteur de CHK-1, une augmentation de la quantité de CHK-1 phosphorylée dans le deuxième échantillon par rapport au premier échantillon indiquant que l'inhibiteur de CHK-1 inhibe sa cible moléculaire CHK-1.

2. Méthode selon la revendication 1, **caractérisée en ce que** l'échantillon est une tumeur, le sang périphérique, une cellule obtenue par grattage, un follicule pileux, une biopsie par poinçon de la peau ou un échantillon buccal.

3. Méthode selon l'une ou l'autre des revendications 1 et 2, **caractérisée en ce que** la CHK-1 phosphorylée est déterminée par un anticorps contre la CHK-1.

4. Méthode selon l'une quelconque des revendications 1-3, **caractérisée en ce que** la CHK1 phosphorylée est phosphorylée au niveau de Ser 345.

5. Méthode pour déterminer si un inhibiteur de CHK-1 inhibe sa cible moléculaire CHK-1, la méthode comprenant:
(a) la détermination de la présence d'H2AX phosphorylé dans un premier échantillon provenant d'un patient atteint de cancer à tester qui a reçu un agent endommageant l'ADN, et
(b) la détermination de la présence d'H2AX phosphorylé dans un deuxième échantillon provenant d'un patient atteint de cancer à tester qui a reçu un agent endommageant l'ADN et un inhibiteur de CHK-1, une augmentation de la présence d'H2AX phosphorylé dans le deuxième échantillon par rapport au premier échantillon indiquant qu'un inhibiteur de CHK-1 inhibe sa cible moléculaire CHK-1.

6. Méthode selon la revendication 5, **caractérisée en ce que** l'échantillon est un prélèvement de sang ayant des cellules tumorales circulantes.

7. Méthode selon l'une quelconque des revendications précédentes, **caractérisée en ce que** le cancer est une tumeur non solide ou une tumeur solide.

8. Méthode selon la revendication 7, **caractérisée en ce que** la tumeur est une leucémie, un myélome multiple ou un lymphome.

9. Méthode selon l'une quelconque des revendications précédentes, **caractérisée en ce que** l'agent endommageant l'ADN est un agent alkylant, un anti-métabolite, un inhibiteur de la topoisomérase, un radiomimétique ou le rayonnement gamma.

10. Méthode selon la revendication 9, **caractérisée en ce que** l'anti-métabolite est le 5-fluorouracile, la fludarabine ou la gemcitabine.
